(19) Europäisches Patentamt
European Patent Office
Office européen des brevets

(11) **EP 3 778 922 A1**

(12) **EUROPEAN PATENT APPLICATION**

(43) Date of publication:
**17.02.2021 Bulletin 2021/07**

(51) Int Cl.:
***C12Q 1/686*** (2018.01)    ***G01N 21/64*** (2006.01)

(21) Application number: **20163228.8**

(22) Date of filing: **16.03.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **11.08.2019   US 201962885303 P**
                **16.10.2019   TW 108137161**

(71) Applicant: **Credo Diagnostics Biomedical Pte. Ltd. Singapore 268802 (SG)**

(72) Inventors:
• **Lai, Ying-Ta**
  **221 New Taipei City (TW)**
• **Ou, Yu-Cheng**
  **221 New Taipei City (TW)**
• **Chen, Yi-Hsi**
  **221 New Taipei City (TW)**
• **Chen, Kuan-Ying**
  **221 New Taipei City (TW)**
• **Chen, Hsi-Yuan**
  **221 New Taipei City (TW)**
• **Liu, Yu-Yu**
  **221 New Taipei City (TW)**

(74) Representative: **Straus, Alexander**
  **2K Patent- und Rechtsanwälte - München Keltenring 9**
  **82041 Oberhaching (DE)**

(54) **REAL-TIME PCR AND FLUORESCENCE SPECTROMETRY**

(57) An analytical method includes obtaining a first data and performing curve fitting on the first data according to at least one basis (BS1 to BS3) to generate at least one coefficient corresponding to the at least one basis (BS1 to BS3). A function pattern corresponding to one of the at least one basis (BS1 to BS3) has asymmetry.

FIG. 6

**EP 3 778 922 A1**

## Description

Field of the Invention

The present invention relates to an analytical system and analytical method, and more particularly, to an analytical system and analytical method of high analysis accuracy and high analysis efficiency, and beneficial to miniaturization.

Background of the Invention

Deoxyribonucleic acid (DNA) amplification reaction of Polymerase chain reaction (PCR) is a crucial technology in molecular biology. In real-time PCR, DNA amplification and quantitative analysis of DNA amplification reaction are performed simultaneously in the same analyte container. In the quantitative analysis after DNA amplification, fluorescent signals excited by the analyte are separated into different optical paths, and the signals of different frequency bands filtered by band pass filters are then analyzed. However, signals in adjacent frequency bands may cause crosstalk, which may vary with solution concentration or temperature, thereby affecting analysis accuracy and analysis efficiency. Furthermore, excitation efficiency to excite the analyte with xenon light of wideband is low. Xenon light of wideband is thus not advantageous to the detection of an analyte of low concentration or the detection of various analytes. The optical system of xenon light of a xenon lamp is complicated and bulky.

Summary of the Invention

It is therefore an objective of the present invention to provide an analytical system and analytical method of high analysis accuracy and high analysis efficiency, and beneficial to miniaturization.

This is achieved by an analytical method according to the independent claim 1 or by an analytical system according to the independent claim 12 here below. The dependent claims pertain to corresponding further developments and improvements.

As will be seen more clearly from the detailed description following below, the present invention discloses an analytical method. The analytical method includes obtaining a first data; and performing curve fitting on the first data according to at least one basis, to generate at least one coefficient corresponding to the at least one basis. A function pattern corresponding to one of the at least one basis has asymmetry.

Another embodiment of the present invention further discloses an analytical system. The analytical system includes a storage device, for storing a program code; and a processing circuit, for executing the program code. An analytical method compiled into the program code includes obtaining a first data; and performing curve fitting on the first data according to at least one basis, to generate at least one coefficient corresponding to the at least one basis. A function pattern corresponding to one of the at least one basis has asymmetry.

Brief Description of the Drawings

FIG. 1 is a schematic diagram of an analytical system according to an embodiment of the present invention.
FIG. 2 is a flow chart of an analytical method according to an embodiment of the present invention.
FIG. 3 is a schematic diagram of a noise removal step of the analytical method of FIG. 2 according to an embodiment of the present invention.
FIG. 4 is a schematic diagram of a partial extraction step of the analytical method of FIG. 2 according to an embodiment of the present invention.
FIG. 5 is a schematic diagram of bases adopted in the analytical method of FIG. 2 according to an embodiment of the present invention.
FIG. 6 is a schematic diagram of a curve fitting step of the analytical method of FIG. 2 according to an embodiment of the present invention.
FIG. 7 is a schematic diagram of an analytical system according to an embodiment of the present invention.

Detailed Description

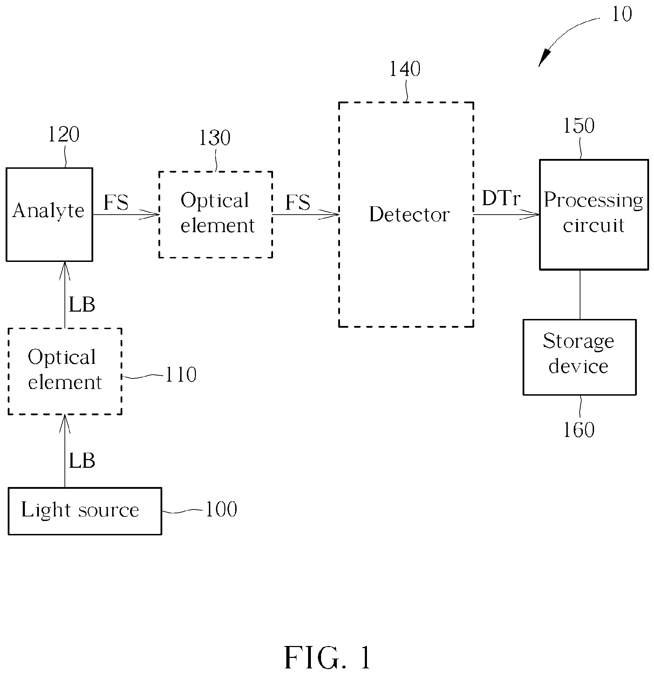

Please refer to FIG. 1, which is a schematic diagram of an analytical system 10 according to an embodiment of the present invention. The analytical system 10 is utilized to detect and analyze fluorescent signals in real time. The analytical system 10 includes a light source 100, optical elements 110, 130, an analyte 120, a detector 140, a processing circuit 150, and a storage device 160. A light beam LB emitted by the light source 100 is directed by the optical element 110 to the analyte 120, and the analyte 120 is excited to generate a fluorescent signal FS (also referred to as a first fluorescent signal). The fluorescent signal FS may be directed to the detector 140 by the optical component 130. The detector 140 receives the fluorescent signal FS and measures a relation between (light) intensity and a wavelength of the fluorescent signal FS, and outputs data DTr measured by itself to the processing circuit 150. The data DTr may be spectral data. The processing circuit 150 may analyze the spectral data transmitted from the detector 140 according to an analytical method compiled into a program code. The storage device 160 may be configured to store the spectral data, the program code of the analytical method or other information.

Briefly, the analyte 120 may include at least one known substance. The light beam LB emitted by the light source 100 may excite the known substance in the analyte 120, such that the fluorescent signal FS is emitted.

Since relation between wavelength and (light) intensity of a fluorescent signal (also referred to as the second fluorescent signal) excited from each known substance is known, the processing circuit 150 may analyze the relation between the wavelength and the (light) intensity of the fluorescent signal FS excited from the analyte 120 so as to determine concentration of each known substance in the analyte 120.

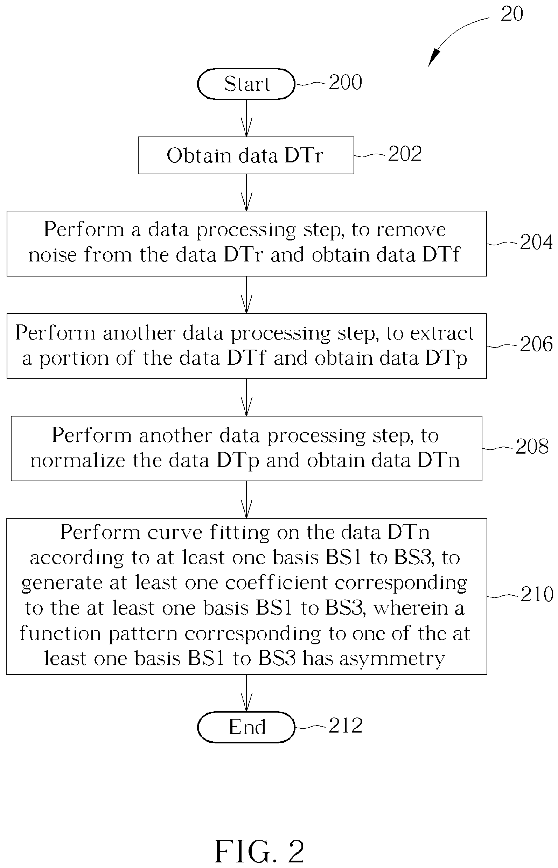

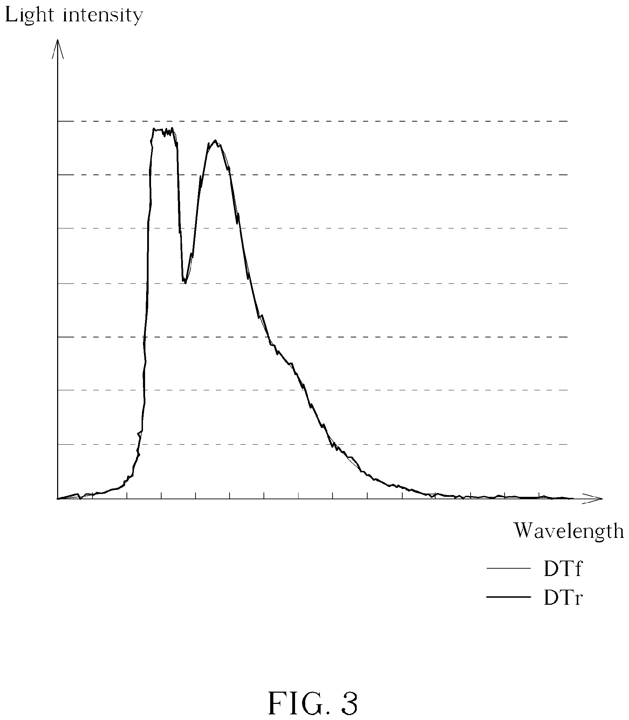

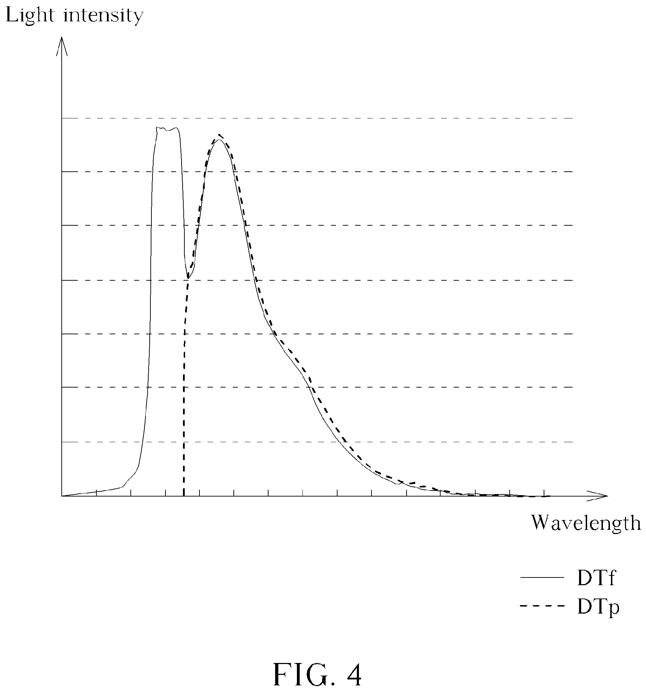

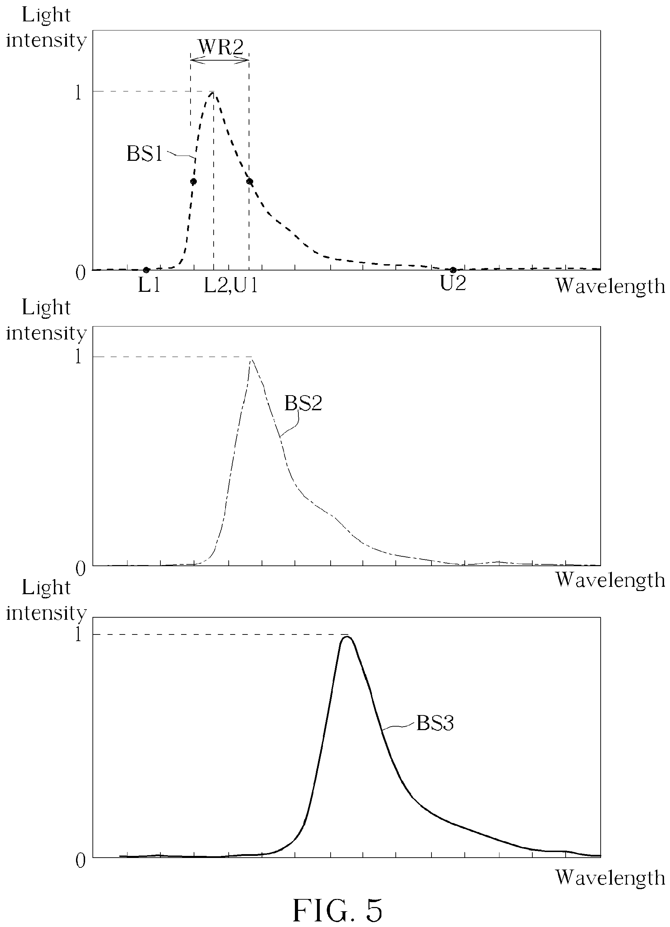

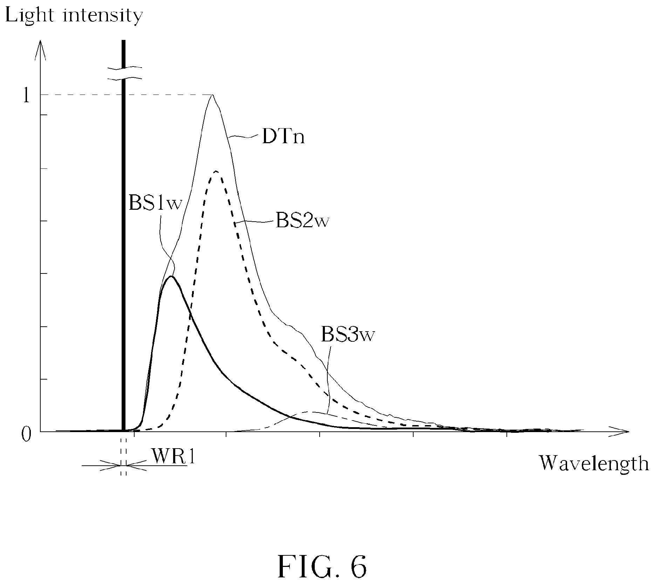

Furthermore, please refer to FIG. 2 to FIG. 6 together. FIG. 2 is a flow chart of an analytical method 20 according to an embodiment of the present invention. FIG. 3 is a schematic diagram of a noise removal step of the analytical method 20 of FIG. 2 according to an embodiment of the present invention. FIG. 4 is a schematic diagram of a partial extraction step of the analytical method 20 of FIG. 2 according to an embodiment of the present invention. FIG. 5 is a schematic diagram of bases BS1 to BS3 adopted in the analytical method 20 of FIG. 2 according to an embodiment of the present invention. FIG. 6 is a schematic diagram of a curve fitting step of the analytical method 20 of FIG. 2 according to an embodiment of the present invention. The analytical method 20 may be compiled into a program code and executed by the processing circuit 150 of FIG. 1. The analytical method 20 may include steps as follows:

Step 200: Start.
Step 202: Obtain data DTr.
Step 204: Perform a data processing step, to remove noise from the data DTr and obtain data DTf.
Step 206: Perform another data processing step, to extract a portion of the data DTf and obtain data DTp.
Step 208: Perform another data processing step, to normalize the data DTp and obtain data DTn.
Step 210: Perform curve fitting on the data DTn according to at least one basis BS1 to BS3, to generate at least one coefficient corresponding to the at least one basis BS1 to BS3, wherein a function pattern corresponding to one of the at least one basis BS1 to BS3 has asymmetry.
Step 212: End.

Specifically, in step 202, the processing circuit 150 receives the data DTr transmitted by the detector 140. The data DTr may be spectral data, which involves a relation between the (light) intensity and the wavelength of the fluorescent signal FS of the analyte 120 excited by the light beam LB.

Step 204 may involve a noise removal step to improve subsequent data analysis accuracy. That is, the processing circuit 150 may perform a data processing step to remove noise (for example, background noise) from the data DTr and convert the data DTr into the data DTf. In some embodiments, the processing circuit 150 may perform noise elimination algorithm in a time domain or a frequency domain. In some embodiments, noise elimination may be performed by a low pass filter, but is not limited thereto. In some embodiments, noise elimination may be performed by a hardware filter. Alternatively, noise elimination algorithm may be performed by a software filter to achieve noise elimination, but not limited thereto. As shown in FIG. 3, a function pattern corresponding to the data DTf is a smoother curve if compared to a function pattern corresponding to the data DTr.

Step 206 may involve a partial extraction step to reduce the interference caused by the light beam LB emitted from the light source 100 or to avoid (or get rid of) interference bands of other light sources. That is, the processing circuit 150 may perform a data processing step to extract a portion of wavelength intervals of the data DTf and convert the data DTf into the data DTp. Therefore, the processing circuit 150 may analyze spectral data for all wavelength intervals or specific wavelength intervals. In some embodiments, the partial extraction step may be performed by hardware or software, but is not limited thereto. As shown in FIG. 4, the function pattern corresponding to the data DTf overlaps a function pattern corresponding to the data DTp only on the right hand side.

Step 208 may involve a normalization step to improve subsequent data analysis efficiency. That is, the processing circuit 150 may perform a data processing step to normalize the data DTp and convert the data DTp into the data DTn. In some embodiments, the processing circuit 150 may perform a normalizing algorithm on the (light) intensity of the data DTp - for instance, maximum (light) intensity may be normalized. In some embodiments, normalization may be expressed as $DTn(\lambda)=(DTp(\lambda)-min(DTp))/(max(DTp)-min(DTp))$, where $max(DTp)$ is the maximum (light) intensity value of the data DTp, and $min(DTp)$ is the minimum (light) intensity value of the data DTp. In addition, $DTp(\lambda)$ and $DTn(\lambda)$ are corresponding (light) intensity values of the data DTp and DTn at a specific wavelength respectively.

In step 210, the processing circuit 150 may perform curve fitting on the data DTn (also referred to as the first data) according to the bases BS1 to BS3 to generate coefficients in the bases BS1 to BS3. The bases BS1 to BS3 are spectral data respectively. The bases BS1 to BS3 respectively provide a relation between wavelength and (light) intensity of a fluorescent signal (also referred to as a second fluorescent signal) from one known substance being excited. As shown in FIG. 6, data components BS1w to BS3w respectively represent a relation between wavelength and (light) intensity of a fluorescent signal from a known substance in the analyte 120 being excited. A sum of light intensities of the data components BS1w to BS3w at a specific wavelength is equal to the (light) intensity of the data DTn at the wavelength. A ratio of the data component BS1w (or the data components BS2w, BS3w) to the basis BS1 (or the bases BS2, BS3) is a coefficient corresponding to the basis BS1 (or the bases BS2, BS3). In other words, $DTn(\lambda)=BS1w(\lambda)+BS2w(\lambda)+BS3w(\lambda)=c1*BS1(\lambda)+c2*BS2(\lambda)+c3*BS3(\lambda)$, where $BS1w(\lambda)$ to $BS3w(\lambda)$ are the (light) intensity values corresponding to the data components BS1w to BS3w at a specific wavelength respectively. $BS1(\lambda)$ to $BS3(\lambda)$ are the (light) intensity values corresponding to the bases BS1 to BS3 at a specific wavelength respectively. Besides, c1 to c3 are the coefficients corresponding to the bases BS1 to BS3 respectively. The processing circuit 150 determines the relation (namely, the data components BS1w to BS3w as shown in FIG. 6) between the wavelength and the (light) intensity of the fluorescent signal from each known substance in the analyte 120 excited by the light beam LB according to the coefficients corresponding to the bases BS1 to BS3 so as to determine concentration of each of the known substances in the analyte 120.

By step 210, even if distances between the data components BS1w to BS3w are relatively close (for example, when a difference between wavelengths corresponding to local maximums of any two of the data components BS1w to BS3w is small or when crosstalk is severe), even if a difference in excitation efficiency of any two different known substances is large, or even if concentration or excitation efficiency of a known substance is low, the data components BS1w to BS3w of each known substance may be effectively analyzed. In some embodiments, a real-time polymerase chain reaction (PCR) occurs in the analyte 120, and the processing circuit 150 determines the concentration of each known substance in the analyte 120 so as to calculate the amount after DNA amplification. The fluorescent signal FS may be the fluorescent signal excited by the reagent (bonded to two strands of DNA) of the analyte 120 in a DNA amplification reaction of a real-time PCR. In this case, the amount after DNA amplification may be calculated according to the coefficients corresponding to the bases BS1 to BS3.

In some embodiments, the curve fitting is performed according to a least square fit method, a multiple linear regression method, a principal component analytical method, a point-wise cross-correlation method, and a least absolute deviation regression method or a wavelet transform method. In some embodiments, the (light) intensities of the bases BS1 to BS3 have been normalized individually; for instance, the maximum (light) intensity of each of the bases BS1 to BS3 is normalized. In some embodiments, as shown in FIG. 5, the function pattern corresponding to the basis BS1 (or the bases BS2, BS3) may have asymmetry. In some embodiments, as shown in FIG. 5, the definite integral of the function pattern of the basis BS1 (or the bases BS2, BS3) in a first wavelength interval may be smaller than the definite integral of the function pattern in a second wavelength interval. It may be expressed as:

$$\int_{L1}^{U1} BS1(\lambda)d\lambda < \int_{L2}^{U2} BS1(\lambda)\, d\lambda$$

where L1 is an (integral) lower limit of the first wavelength interval and corresponds to a first low intensity point of the function pattern. U2 is an (integral) upper limit of the second wavelength interval and corresponds to a second low intensity point of the function pattern. The function value of the low intensity points may be lower than the noise level or the detection limit of the spectrometer. U1 and L2 are an (integral) upper limit of the first wavelength interval and an (integral) lower limit of the second wavelength interval respectively. Both U1 and L2 correspond to a local maximum of the function pattern. In some embodiments, the function pattern corresponding to the basis BS1 (or the bases BS2, BS3) may have more than one local maximums. In some embodiments, the functional pattern corresponding to the basis BS1 (or the bases BS2, BS3) may be determined by experiments or theoretical calculations, for instance, measuring the excitation spectrum or the emission spectrum of a specific known substance.

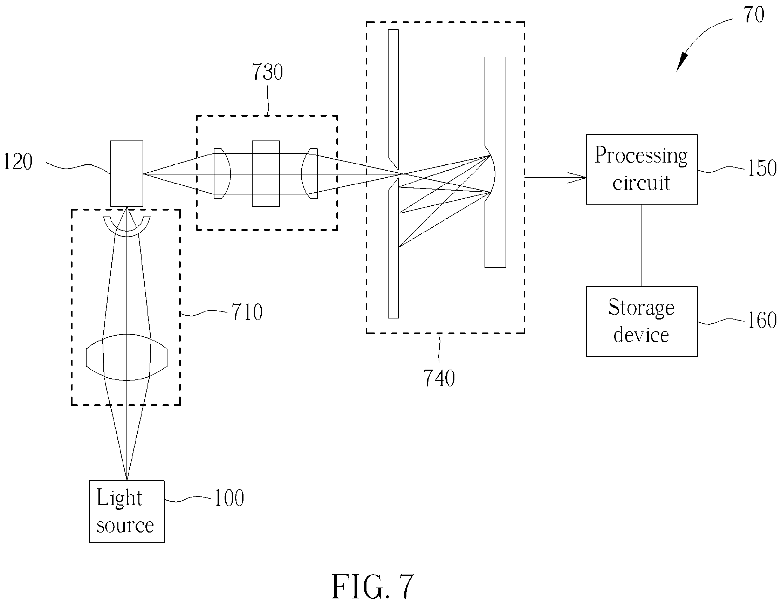

It is noteworthy that the analytical system 10 shown in FIG. 1 is an exemplary embodiment of the present invention, and those skilled in the art may readily make alterations and modifications. For example, please refer to FIG. 7, which is a schematic diagram of an analytical system 70 according to an embodiment of the present invention. The structure of the analytical system 70 is similar to that of the analytical system 10. Therefore, the same numerals and notations denote the same components in the following description, and the similar parts are not detailed redundantly. The optical components 110, 130 and the detector 140 of the analytical system 10 may be implemented as optical components 710, 730 and a detector 740 of the analytical system 70.

Specifically, the light source 100 may be a light source of high (light) intensity and narrow band. As a result, even if the distances between the data components BS1w to BS3w are relatively close (for example, when a difference between the wavelengths corresponding to the local maximums of any two of the data components BS1w to BS3w is small or when crosstalk is severe), even if a difference in excitation efficiency of any two different known substances is large, or even if concentration or excitation efficiency of a known substance is low, each known substance may be effectively excited. For example, in some embodiments, (light) intensity of the light source 100 may range from 10 milliwatts (mW) per square millimeter (millimeter, $mm^2$) to 500 milliwatts per square millimeter (namely, 500 $mW/mm^2$). Therefore, the light source 100 is a source of relatively high (light) intensity. In some embodiments, (instantaneous) output power of the light source 100 may be in a range of 1 milliwatts (mW) to 500 milliwatts, but is not limited thereto.

In some embodiments, a wavelength range (or bandwidth) of the light source 100 is less than one tenth of the wavelength range (or bandwidth) of the fluorescent signal from the at least one known substance being excited. Therefore, the light source 100 is a source of relatively narrow band. The wavelength range (or bandwidth) may be defined by a full width at half maximum (FWHM). In some embodiments, as shown in FIG. 6, a wavelength range WR1 of the light source 100 is smaller than a wavelength range WR2 of the basis BS1 (or the bases BS2, BS3). For example, the wavelength range WR1 of the light source 100 is smaller than one tenth of the wavelength range WR2 of the basis BS1 (or the bases BS2, BS3). In some embodiments, the wavelength range of the light source 100 may range from 0.1 nanometers (nm) to 2 nanometers, but is not limited thereto. In some embodiments, the light source 100 may be a laser light source. Furthermore, the light source 100 may be a diode laser light source or a semiconductor laser light source, such as a Fabry-Perot (FP) semiconductor laser, a single-frequency semiconductor laser, a distributed feedback laser (DFB) or a vertical-cavity surface-emitter laser (VCSEL), but is not limited to these. In some embodiments, the light source 100 may be a semiconductor laser light source set including one or more semiconductor laser light sources, and all or part of the semiconductor laser light sources may have different central wavelengths. In some embodiments, the light source 100 may be a light-emitting diode light source having a radiation angle less than or equal to 10 degrees, but is not limited thereto. In some embodiments, the light source 100 may be a light-emitting diode light source set including one or more light-emitting diode light sources, and all or part of the light-emitting diode light sources may have different central wavelengths. In some embodiments, a central wavelength of the light source 100 may be in a range of 405 nanometers to 660 nanometers, but is not limited thereto.

The analyte 120 may include at least one reactant or reagent, and the reagent may include at least one fluorescent probe or fluorescent dye, but is not limited thereto. In some embodiments, the fluorescent probe or fluorescent dye may be FAM, VIC, HEX, ROX, CY3, CY5, CY5.5, JOE, TET, SyBR, Texas Red, TAMRA, NED, Quasar 705, Alexa488, Alexa546, Alexa594, Alexa633, Alexa643, Alexa680 or other fluorescent probes or fluorescent dyes. In some embodiments, a central wavelength of the fluorescent probe or the fluorescent dye is in a range of 340 nanometers to 850 nanometers, but is not limited thereto. In addition, the analyte 120 may be kept in an analyte container.

The optical elements 710 and 730 may be utilized to adjust beam width of a light beam, for example, to converge or to diverge the light beam. Alternatively, the optical elements 710 and 730 may be further utilized to adjust beam direction of the light beam. In some embodiments, the optical element 710 or 730 may include at least one convergent lens, but is not limited thereto. In some embodiments, the optical element 710 or 730 may include at least one of a biconvex lens, a plano-convex lens, a dual lens, an aspherical lens, an achromatic lens, an aberration lens, a Fresnel lens, a plano-concave lens, a biconcave lens, a positive/negative meniscus lens, an axon prism, a gradient (refractive) index lens, a micro-lens array, a lenticular lens, a diffractive optical element, a parabolic mirror, a holographic optical element, or an optical waveguide element. In some embodiments, the optical element 710 or the optical element 730 may be removed from or omitted in the analytical system 70.

In some embodiments, the detector 740 may be a spectrometer. In some embodiments, the detector 740 may be a miniature spectrometer to facilitate miniaturization of the analytical system 70. For example, the detector 740 is a Micro-Electromechanical Systems (MEMS) based spectrometer, but is not limited thereto. In some embodiments, a wavelength range of the detector 740 may range from 340 nanometers to 850 nanometers, but is not limited thereto.

The processing circuit 150 may analyze the spectral data transmitted from the detector 140 according to the analytical method 20 compiled into the program code. In some embodiments, the processing circuit 150 may be a central processing unit (CPU), a microprocessor, or an application-specific integrated circuit (ASIC). In some embodiments, the processing circuit 150 may control the light source 100 to be turned on or off during predetermined time interval(s). In addition, when the light source 100 is turned on, the detector 140 synchronously detects the fluorescent signal FS. In some embodiments, the processing circuit 150 may control the light source 100 to be turned on or off during predetermined time interval(s) by manipulating a working current of the light source 100 or by controlling an optical path switch. The optical path switch may be a mechanical optical path switch or an electronic optical path switch.

In some embodiments, the storage device 160 may be any data storage device (or circuit) for storing a program code. The processing circuit 150 may read and execute the program code via the storage device 160. For example, the storage device 160 may be a subscriber identity module (SIM), a read-only memory (ROM), a flash memory, a random access memory (RAM), a hard disk, an optical data storage device, a non-volatile storage device, a non-transitory computer-readable medium, and is not limited to these.

Besides, the analytical method 20 is an exemplary embodiment of the present invention, and those skilled in the art may readily make alterations and modifications. For example, the step 204, 206, or 208 in the analytical method 20 may be optional. In the case of low noise, the step 204 in the analytical method 20 may be omitted, and a portion of the data DTr may be extracted to obtain the data DTp in step 206. In the absence of interference from other light sources, the step 206 in the analytical method 20 may be omitted, and a portion of the data DTf may be extracted to obtain the data DTn in step 208. If normalization is dispensable, the step 208 in the analytical method 20 may be omitted, and the curve fitting may be performed on the data DTp in step 210. Similarly, when the step 204 or the step 206 is omitted, the curve fitting may be performed on the data DTr or the data DTf. In addition, the order of step 204, step 206, or step 208 may be interchangeable.

To sum up, the present invention does not adopt a xenon lamp of wide band, but uses the light source 100 of high intensity and narrow band instead. Therefore, the present invention may detect the analyte 120 of low concentration, and reduce complexity and size of the analytical system 10 to meet requirements of detection in real time. In addition, the present invention does not separate the fluorescent signal FS excited from the analyte 120 into different optical paths and does not filter the fluorescent signal FS in different optical paths into signals in different frequency bands. Instead, the present invention performs curve fitting on the data DTn corresponding to the fluorescent signal FS to generate the coefficients corresponding to the bases BS1 to BS3 in order to determine the relation (namely, the data components BS1w to BS3w) between the wavelength and the (light) intensity of the fluorescent signal from each known substance in the excited analyte 120, thereby determining the concentration of each known substance in the analyte 120. As a result, even if crosstalk occurs, or even if a difference of excitation efficiencies of any two different known substances is large, each known substance may still be excited and analyzed effectively, thereby improving analysis accuracy and analysis efficiency. In addition, the present invention may improve signal-to-noise ratio (SNR) by means of data processing step(s).

**Claims**

1. An analytical method, **characterised by,** comprising:

    obtaining a first data (DTr, DTf, DTp, DTn); and
    performing curve fitting on the first data (DTr, DTf, DTp, DTn) according to at least one basis (BS1 to BS3), to generate at least one coefficient corresponding to the at least one basis (BS1 to BS3), wherein a function pattern corresponding to one of the at least one basis (BS1 to BS3) has asymmetry.

2. The analytical method of claim 1, **characterised in that** the first data (DTn) is a relation between an intensity and a wavelength of a first fluorescent signal (FS) from an analyte (120) excited, and each of the at least one basis (BS1 to BS3) is a relation between an intensity and a wavelength of a second fluorescent signal from a known substance excited respectively.

3. The analytical method of claim 1, **characterised in that** the definite integral of the function pattern in a first wavelength interval is less than the definite integral of the function pattern in a second wavelength interval, a lower limit (L1) of the first wavelength interval corresponds to a first low intensity point of the function pattern, an upper limit (U2) of the second wavelength interval corresponds to a second low intensity point of the function pattern, and both an upper limit (U1) of the first wavelength interval and a lower limit (L2) of the second wavelength interval correspond to a local maximum of the function pattern.

4. The analytical method of claim 1, **characterised in that** the curve fitting is performed according to a least square fit method, a multiple linear regression method, a principal component analytical method, a point-wise cross-correlation method, and a least absolute deviation regression method or a wavelet transform method.

5. The analytical method of claim 1, **characterised by,** further comprising:

    obtaining a second data (DTr); and
    performing a data processing step, to remove noise from the second data (DTr) and obtain the first data (DTf).

6. The analytical method of claim 1, **characterised by,** further comprising:

    obtaining a second data (DTf); and
    performing a data processing step, to extract a portion of the second data (DTf) and obtain the first data (DTp).

7. The analytical method of claim 1, **characterised by,** further comprising:

    obtaining a second data (DTp); and
    performing a data processing step, to normalize the second data (DTp) and obtain the first data (DTn).

8. The analytical method of claim 1, **characterised by,** further comprising:
    directing a light beam (LB) emitted from a light source (100) to an analyte (120), to obtain the first data (DTr) by measuring a first fluorescent signal (FS) from the analyte (120) excited.

9. The analytical method of claim 8, **characterised in that** the light source (100) is a laser light source, the bandwidth of the light source (100) is less than one tenth of the bandwidth of one of the at least one basis (BS1 to BS3), and the bandwidth of the light source (100) is defined by a full width at half maximum.

10. The analytical method of claim 8, **characterised in that** the light source (100) is a light-emitting diode (LED) light source of a radiation angle less than or equal to 10 degrees.

11. The analytical method of claim 8, **characterised in that** a real-time polymerase chain reaction is performed on the analyte (120).

12. An analytical system, **characterised by,** comprising:

> a storage device (160), for storing a program code; and
> a processing circuit (150), for executing the program code, wherein an analytical method of any one of the preceding claims 1 to 11 is compiled into the program code.

10

140

120

130

150

Analyte — FS → Optical element — FS → Detector — DTr → Processing circuit

LB

Optical element ~ 110

Storage device

160

LB

Light source ~ 100

FIG. 1

Start —~200

Obtain data DTr —~202

Perform a data processing step, to remove noise from the data DTr and obtain data DTf —~204

Perform another data processing step, to extract a portion of the data DTf and obtain data DTp —~206

Perform another data processing step, to normalize the data DTp and obtain data DTn —~208

Perform curve fitting on the data DTn according to at least one basis BS1 to BS3, to generate at least one coefficient corresponding to the at least one basis BS1 to BS3, wherein a function pattern corresponding to one of the at least one basis BS1 to BS3 has asymmetry —~210

End —~212

20

FIG. 2

Light intensity

Wavelength

—— DTf
—— DTr

FIG. 3

FIG. 4

FIG. 5

Light intensity

FIG. 6

FIG. 7

EP 3 778 922 A1

Europäisches
Patentamt

European
Patent Office

Office européen
des brevets

# EUROPEAN SEARCH REPORT

Application Number

EP 20 16 3228

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2007/051610 A1 (ROCHE DIAGNOSTICS GMBH [DE]; HOFFMANN LA ROCHE [CH] ET AL.) 10 May 2007 (2007-05-10) * the whole document * | 1-12 | INV. C12Q1/686 G01N21/64 |
| X | US 2018/088029 A1 (ASOGAWA MINORU [JP]) 29 March 2018 (2018-03-29) | 1-10,12 | |
| Y | * paragraphs [0006], [0024], [0030] - [0034], [0051] - [0054]; figure 2 * | 11 | |
| X | US 2003/223059 A1 (LI QINGBO [US]) 4 December 2003 (2003-12-04) | 1-10,12 | |
| Y | * paragraphs [0060], [0075], [0097] - [0099]; figure 13 * | 11 | |
| Y | LEE ET AL: "A novel real-time PCR machine with a miniature spectrometer for fluorescence sensing in a micro liter volume glass capillary", SENSORS AND ACTUATORS B: CHEMICAL, ELSEVIER BV, NL, vol. 100, no. 3, 15 May 2004 (2004-05-15), pages 401-410, XP005135407, ISSN: 0925-4005, DOI: 10.1016/J.SNB.2004.02.012 | 11 | |
| A | * page 409, column 2, paragraph 4 - page 410, column 1 * | 1-10,12 | TECHNICAL FIELDS SEARCHED (IPC) G01N C12Q |

The present search report has been drawn up for all claims

| Place of search | Date of completion of the search | Examiner |
|---|---|---|
| Munich | 6 October 2020 | Huenges, Alexandra |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 20 16 3228

This annex lists the patent family members relating to the patent documents cited in the above-mentioned European search report.
The members are as contained in the European Patent Office EDP file on
The European Patent Office is in no way liable for these particulars which are merely given for the purpose of information.

06-10-2020

| Patent document cited in search report | | Publication date | Patent family member(s) | | Publication date |
|---|---|---|---|---|---|
| WO 2007051610 | A1 | 10-05-2007 | CA | 2627914 A1 | 10-05-2007 |
| | | | CN | 101300478 A | 05-11-2008 |
| | | | EP | 1946081 A1 | 23-07-2008 |
| | | | JP | 2009515153 A | 09-04-2009 |
| | | | US | 2007098594 A1 | 03-05-2007 |
| | | | WO | 2007051610 A1 | 10-05-2007 |
| US 2018088029 | A1 | 29-03-2018 | JP | 6521056 B2 | 29-05-2019 |
| | | | JP | WO2016157270 A1 | 25-01-2018 |
| | | | US | 2018088029 A1 | 29-03-2018 |
| | | | WO | 2016157270 A1 | 06-10-2016 |
| US 2003223059 | A1 | 04-12-2003 | AU | 4923701 A | 24-09-2001 |
| | | | US | 2003223059 A1 | 04-12-2003 |
| | | | WO | 0169211 A1 | 20-09-2001 |

EPO FORM P0459

For more details about this annex : see Official Journal of the European Patent Office, No. 12/82